# EUROPEAN PATENT APPLICATION

(11) **EP 4 119 123 A1**
(43) Date of publication of application: **18.01.2023**
(21) Application number: 21185637.2
(22) Date of filing: 14.07.2021
(51) Int. Cl.: A61K 9/00, A61K 9/70, A61K 47/36, A61K 31/485

(54) **ORALLY DISINTEGRATING FILM COMPOSITION COMPRISING BUPRENORPHINE**

(71) Applicant: G.L. Pharma GmbH, 8502 Lannach (AT)
(72) Inventor: WACHTER, Christof, 8502 Lannach (AT); KÜHBERGER, Elisabeth, 8502 Lannach (AT); BEA, Manuel, 88499 Altheim (DE); MITTER, Sabrina Janine, 88410 Bad Wurzach (DE); HIESTAND, Bianca, 88444 Ummendorf (DE); WAGNER, Wolfgang, 89233 Neu-Ulm (DE); BECKERT, Thomas, 88447 Warthausen (DE)
(74) Representative: SONN Patentanwälte OG

(57) **Abstract**

Disclosed is an orally disintegrating film composition comprising Buprenorphine as active ingredient and a polymeric matrix, which is comprising of a combination of at least two water-soluble film forming polymers to form a polymeric matrix and wherein Buprenorphine is uniformly dispersed as particles within the polymeric matrix.

## Description

### Field of the Invention

The present invention relates to film delivery systems, especially for oral delivery, which can be formed during manufacture in the form of film strips or sheets and subsequently cut into uniform dosage units, each dosage unit being uniform in content and having distributed Buprenorphine uniformly dispersed within the polymeric matrix comprising of a mixture of at least two bioedible water-soluble polymers. The combination of these polymers enables a precise control of the chemical, physical and mechanical properties of the orally disintegrating film and allows the manufacturing of products with the various desired functionalities. Buprenorphine is dispersed as solid particles in the polymeric matrix.

### Background of the Invention

The route of administration is well known to affect the absorption, distribution, and metabolism parameters of pharmaceutical active ingredients. Drug delivery via intravenous (i.v.) injection is known to permit relatively rapid onset of therapeutic effects, however, i.v. injection is not very practical outside of a clinical setting. Accordingly, the oral route of drug administration is the most suitable and preferred route due to its ease of administration, non-invasiveness, adaptability and most important patient compliance and acceptability, although it typically has a very slow onset of therapeutic effects, and drug potency can be lost due to action of the digestive system and/or first pass metabolism. The oral administration of medicinal and therapeutic agents that occur by absorption across the buccal or sublingual mucosa is an attractive alternative to standard oral administration by ingestion into the gastro-intestinal tract, as it can bypass first pass metabolism in the liver as well as degradation in the digestive tract. By the novel approach of fast dissolving drug delivery systems, for example orally disintegrating films (ODF), a beneficial mucosal administration of drugs can be achieved leading to higher control of the actual dosage of the active ingredient and increased patient compliance especially in pediatrics or geriatrics. ODFs are typically the size of a regular postage stamp and disintegrate on a patient's tongue in a matter of seconds for the rapid release of one or more active ingredients. The formulation of dissolvable films is customarily facilitated through aqueous polymer matrices that span a wide molecular weight (MW) range enabling the rapid dissolution and fast release of the active pharmaceutical agent upon immediate hydration by saliva in the oral cavity and thereby providing flexibility to achieve certain physical properties.

In comparison to other oral dosage forms, orally disintegrating films have several special advantages such as a rapid disintegrating and dissolution in the oral cavity caused by a larger surface area which improves the onset of action, lower the dosing, and enhance the efficacy and safety profile of the medicament. Since the oral or buccal mucosa being highly vascularized, drugs can be absorbed directly and can enter the systemic circulation without undergoing first-pass hepatic metabolism enabling a reduction of the dose of the active ingredient concomitant with a minimization of associated side-effects. This feature of orally disintegrating films can be exploited in preparing products with improved oral bioavailability of active ingredients that undergo strong first pass effects.

Furthermore, the administered dose of an orally disintegrating film is precisely controlled through the size of the film strip, because the active ingredient is uniformly distributed in the polymer matrix of the film. Moreover, orally disintegrating films are easy in handling transportation and storage and are not as fragile as e.g. orally disintegrating tablets. Besides, the orally disintegrating film dosage form is preferable for patients suffering from dysphagia, repeated emesis, motion sickness, and mental disorders as they are unable to swallow large quantity of water. Therefore, important practical applications for administration via orally disintegrating films include emergency care situations where rapid administration of drugs by non-skilled personnel could be life-saving; in unconscious patients who may have overdosed or experienced a seizure; in elderly dementia patients with dysphagia; as a facile and convenient route of administration of medication to young children; and for drug delivery of medication to animals.

As an illustration, there are several patent publications, for example EP 1 463 491 A1 (WO 03/030882 A1), EP 2 371 358 A1, EP 3 160 589 A1 (WO 2015/200233 A1), EP 3 106 151 A1, EP 3 106 152 A1, EP 3 452 025 A1 (WO 2017/180707 A1) and WO 2020/014776 A1, that describe application of ODF technology used for the mucosal drug delivery of various pharmaceutical agents by incorporating them in a bio-edible polymeric matrix. However, the orally disintegrating film compositions enclosed in these patents are mainly based on one single film-forming polymer, e.g. pullulan (*cf.* EP 3 452 025 A1, WO 2020/014776 A1) or cellulose derivatives, such as hydroxypropyl cellulose or hydroxypropylmethyl cellulose (*cf.* EP1 463 491 A1, EP 3 160 589 A1), and the properties of the matrix are modified only by using different plasticizers or additives. However, films made exclusively from one polymeric component have low flexibility and suffer from the limitation that in order to put higher drug levels in the film an increased polymer content is necessary to preserve uniformity of the film.

Further developments in the field show that several properties of a thin-film, such as flexibility, tensile strength and overall structural integrity, can be easily varied by the application of a combination of different water-soluble polymers for the formation of the film-matrix.

For example, in patent application EP 1 463 490 A1, to Yang et al., ingestible water-soluble film compositions comprising of a glucan and a water-soluble polymer, preferably modified cellulosic materials, is disclosed. By reducing the content of glucan in said disintegrating films, higher amounts of active components can be incorporated and higher structural integrity was achieved compared to conventional glucan-based films.

Furthermore, patent application EP 1 713 450 A1 (WO 2005/074894 A1), to Singh et al., discloses a rapidly dissolving film having significant drug loading capability while providing sustained and controlled release of an active agent. This film is made up of high molecular weight synthetic polymers (polyvinylpyrrolidone, polyethylene glycol) and contains a rapidly dissolving oligomeric material, such as maltodextrin.

Moreover, patent application EP 2 120 895 A2 (WO 2008/089151 A2) to Myers et al., discloses orally disintegrating films with high amounts (at least 30%) of various pharmaceutical active ingredient and the preparation method of such film strips. In most of the film compositions a combination of polyethylene glycol and polydextrose in various ratios was utilized to facilitate the incorporation of such high dosages with a reasonable uniformity and distribution of active ingredient within the film strip.

The above described features of orally disintegrating film composition are very beneficial for the safe and abuse-proof application of narcotic drugs, like Buprenorphine, which is demonstrated in the present invention. Buprenorphine is a semi-synthetic opioid which is used to treat opioid addiction (substitution therapy) in high dosages (>2 mg) and Buprenorphine is a potent opioid analgesic with a longer duration of action than morphine. For that reason, an opioid receptor antagonist (e.g. naloxone) cannot fully reverse the effects of Buprenorphine. From the prior art, Buprenorphine is only formulated as sublingual tablets for example under the trade name Subutex^{®} which comprise the active agent Buprenorphine hydrochloride for drug substitution therapy. The suitability of particularly Buprenorphine for drug substitution therapy had been recognized early on in view of Buprenorphine's very long elimination half-life (reported as approximately 20 to 37 hours), which allows a reduced frequency of administration. As a consequence, drug addicts who participate in drug substitution therapy have to report less frequently to the medical agency or healthcare professional supervising the substitution program. Furthermore, the sublingual absorption of Buprenorphine has the advantage that an abuse of Buprenorphine is less likely to occur. The tablets that are currently on the market in the form of Subutex^{®} preparations are for sublingual administration and typically disintegrate over a time period of five to ten minutes. However, within that time period the drug addict may be able to divert the tablet before subsequently either selling the tablets on the street or isolating the active agents therefrom. In order to reduce and eliminate these problems, the present invention provides orally disintegrating dosage forms which comprise the active agent Buprenorphine and which release Buprenorphine instantly after oral, preferably sublingual, administration of the drug.

As consequence, there is a strong need for orally disintegrating films containing Buprenorphine as active ingredient with abuse-proof formulations that are easy to manufacture and avoid a substantial number of additives, while at the same time, the desired properties of the ODF such as disintegration times, dissolution times, smooth appearance appropriate for commercialization, strength for withstanding handling, uniform distribution of Buprenorphine, etc. can be controlled by the composition of the formulation. Furthermore, the incorporation of Buprenorphine in these orally disintegrating films with high accuracy of the dosage is highly desirable. This uniform distribution of Buprenorphine can be achieved by a homogenous dispersion of Buprenorphine within an adaptable polymeric matrix comprising of a mixture of two water-soluble polymers.

### Summary of the Invention

According to one aspect of the present invention an orally disintegrating film composition is provided comprising of Buprenorphine as active ingredient and a polymeric matrix, which is comprising of a combination of at least two water-soluble film forming polymers to form a polymeric matrix and wherein Buprenorphine is uniformly dispersed as particles within the polymeric matrix, and wherein the composition has a disintegration time of 180 s or less, preferably of 120 s or less, especially of 90 s or less and/or an ultimate tensile strength of 0.8 N/mm² or below, preferably of 0.5 N/mm² or below, especially of 0.3 N/mm² or below.

According to further aspects of the invention, the orally disintegrating film composition can contain Buprenorphine free base as well as any pharmaceutically acceptable salt thereof, such as the hydrochloride, sulfate, bisulfate, tartrate, nitrate, citrate, bitartrate, phosphate, malate, maleate, hydrobromide, hydroiodide, fumarate, succinate salts and the like.

According to further aspects of the present invention, the orally disintegrating film composition comprises a second active ingredient, such as an opioid antagonist, preferably naloxone or a pharmaceutical acceptable salt thereof.

According to further aspects of the present invention, the orally disintegrating film composition is comprised of a first film-forming polymer which is a starch selected from the group consisting of acorn, arracacha, barley, beans, breadfruit, buckwheat, cassava, chickpea, chestnut, corn, lentil, millet, oat, pea, potato, rice, rye, sago, sorghum, sweet potato, tapioca, wheat and combinations thereof, preferably wherein the starch is a pregelatinized starch, a hydroxypropyl starch or a pregelatinized hydroxypropyl starch, especially a pregelatinized (i.e. warm or cold water dispersible) hydroxypropyl pea starch.

Especially pea starches have advantageous properties which make them specifically useable in the combination formulation according to the present invention, especially in their pregelatinized hydroxypropylated form which provides to the pea starches - despite their high amylose content - a higher paste viscosity, paste clarity and improved properties with respect to the extent of syneresis (Ratnayake et al., Starch 54 (2002), 217-234). Such products are also commercially available, e.g. from Roquette under the name LYCOAT with two grades LYCOAT RS 780 (developing lower viscosity) and LYCOAT RS 720 (developing higher viscosity) or as granular hydroxypropyl starch (LYCOAT NG 720), wherein pregelatinized hydroxypropylated pea starches developing higher viscosities (with a glass transition temperature of at least 45°C at a water content of 20 g of water/100g of wet substance and a glass transition temperature of at least 120°C at a water content of 10g of water/100g of wet substance, measured e.g. by differential scanning calorimetry (DSC) measurement of the differential heat flow between a polymer sample and an inert reference at atmospheric pressure and at 25°C) are specifically preferred. The orally disintegrating film composition may further contain a second film forming polymer selected from the group comprising of glucans, pullulan, dextrin, gelatins, glycogen, methylcellulose, ethylcellulose, hydroxypropylmethylcellulose, hydroxyethylcelullose, cellulose acetate phthalate, hydroxypropyl methylcellulose phthalate, carboxymethyl ethylcellulose, hydroxypropylmethyl cellulose acetate succinate, polyvinyl acetate phthalate, maltodextrin, dextran, hydroxypropyl cellulose, sodium carboxymethyl cellulose, poly(methacrylic acid-co-ethyl acrylate), poly(methacrylic acid-co-methyl methacrylate), polyethyleneoxide, polyethyleneglycol, polyvinylpyrrolidone (PVP), polylactic acid (PLA), poly-L-lactide (PLLA), poly-D-lactide (PLDA), poly(lactic-co-glycolic acid) (PLGA), and mixtures thereof, preferably pullulan (a polysaccharide polymer consisting of maltotriose units, also known as α-1,4-;α-1,6-glucan; three glucose units in maltotriose are connected by an α-1,4 glycosidic bond, whereas consecutive maltotriose units are connected to each other by an α-1,6 glycosidic bond; pullulan (CAS NO.: 9057-02-7; E 1204) is produced from starch by the fungus Aureobasidium pullulans). The preferred viscosity of Pullulan is in the range from 100 mm²/s - 180 mm²/s, preferably 140 - 160 mm²/s, more preferably 140 mm²/s.

According to certain embodiments the ratio of the first film-forming polymer to the second water-soluble polymer in the orally disintegrating film composition is between 10:1 to about 1:10, preferably between 6:1 to about 1:4, more preferably between 4:1 to about 2:3.

The orally disintegrating film may further contain a surfactant and/or cosurfactant, which are used to homogenously disperse Buprenorphine within the polymeric matrix. The surfactant and/or cosurfactant may be selected from the group consisting of polyglycolized glycerides, polyoxyethylene glycerides, polyethylene glycol-fatty acid esters, polyethylene glycol glycerol fatty acid esters, transesterfication products of alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters, polysorbates, poloxamers, phospholipids, lecithin, phosphatidylcholines, phosphatidylethanolamines, phosphatidylinositols, phosphatidylserines, phosphatidic acids, and mixtures thereof.

According to certain embodiments, the orally disintegrating film may additionally include one or more plasticizers, taste masking agents, a flavor agent, a solubilizer, a thickener, a coloring agent, an effervescent agent, an antioxidant, a pH modifying agent, vitamins, minerals, a dietary supplement, or mixtures thereof.

According to certain embodiments, the flavoring and/or taste masking agent of the orally disintegrating film composition is present in an amount up to 10% by weight of the film and is selected from the group comprising any one of kleptose, cyclodextrin, cyclodextrin derivatives, ginger, anise, cinnamon, peppermint, licorice, fruit juice, citric acid, citrate, sweeteners, sucrose, glucose, fructose, mannitol, saccharin, aspartame, sucralose, stevia plant derivatives, honey, or any combination thereof.

According to certain embodiments Buprenorphine is present in the orally disintegrating film composition in amounts of up to about 0.1% to about 50% by weight of the total composition, preferably 1% to about 30%, more preferably 10% to about 20%.

According to certain embodiments the orally disintegrating film composition according to the present invention is essentially free of a gum resin.

According to certain embodiments the orally disintegrating film composition according to the present invention is provided without utilizing an oily medium.

In another aspect of the invention the orally disintegrating film composition according to the present invention consists of a single layer.

In certain embodiments the orally disintegrating film composition according to the present invention has a polymeric backing layer.

According to certain embodiments the orally disintegrating film composition according to the present invention has a thickness of the film in a range from about 0.01 to 0.8 mm.

According to certain embodiments the orally disintegrating film composition according to the present invention comprises Buprenorphine and a film forming agent, having a percentage relative standard deviation of assay of Buprenorphine in a batch of said orally disintegrating film composition of less than 2.5%, preferably less than 1%, more preferably less than 0.5%.

According to another aspect of the invention the orally disintegrating film composition according to the present invention comprises Buprenorphine and a film forming agent, wherein disintegration time in phosphate buffered saline solution at pH 6.8 and 25 °C is determined by the mixture of two hydrophilic polymers and is less than about 10 minutes, preferably less than about 2 minutes, more preferably less than about 1 minute.

In certain embodiments, the mechanical and rheological properties of the orally disintegrating film composition according to the present invention are modified by the variation of the ratio of two hydrophilic polymers within the formulation.

### Brief Description of the Drawings

Figure 1 is a block diagram showing the manufacturing procedure of the orally disintegrating film according to an embodiment of this invention.
Figure 2 is a schematic representation of the casting apparatus and the casting procedure.
Figure 3 is a diagram illustrating the dissolution in saliva pH 6.8 medium of Example 8 containing 2 mg Buprenorphine in one ODF in comparison to Subutex^{®} 2 mg.
Figure 4 is a diagram illustrating the dissolution in saliva pH 6.8 medium of Example 9 containing 2 mg Buprenorphine in one ODF in comparison to Subutex^{®} 2 mg.

### Detailed Description of the Invention

The present disclosure is not limited in terms of the particular embodiments described in this application, which are intended as illustrations of various aspects only. Many modifications and variations can be made without departing from the scope of the invention, as will be apparent to those skilled in the art. Functionally equivalent methods within the scope of the disclosure, in addition to those enumerated herein, will be apparent to those skilled in the art from the following descriptions. Such modifications and variations are intended to fall within the scope of the appended claims. It is also to be understood that the terminology used herein is for the purpose of describing particular embodiments only, and is not intended to be limiting.

As will be understood by one skilled in the art, for any and all purposes, such as in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, etc. As a non-limiting example, each range discussed herein can be readily broken down into a lower third, middle third and upper third, etc. As will also be understood by one skilled in the art, all language such as "up to," "at least," and the like include the number recited and refer to ranges which can be subsequently broken down into subranges as discussed above. Finally, as will be understood by one skilled in the art, a range includes each individual member. Thus, for example, a group having 1-3 units refers to groups having 1, 2, or 3 units.

As used herein, the terms "orally disintegrating film", "orally dispersible film", "orally disintegrating film strip", "film strip" and "film" refer to sheets of variable dimensions comprising a polymeric carrier matrix, and having any shape, including rectangular, square, or other desired shapes. The films described herein are typically thin films with thickness that can range from about 10 microns to about 800 microns, preferably from about 80 to about 600 microns, more preferably from 100 to 300 microns, but may be any desired thickness and size so long as they can be placed comfortably into the oral cavity of the user. Films are a single layer and are typically inherently flexible to enable rapid dissolution in the mouth, permeation through the oral mucosa and entry into the bloodstream via capillaries.

The methods for analyzing the different parameters referred to in the present specification are well available to the person skilled in the art. Preferred methods for determining these parameters are disclosed in the example section. In case of doubt, the parameters are to be interpreted as to be measured according to the methods disclosed in the example section. For example, disintegration time is preferably measured as in the section "Disintegration" in the example section. The rheological properties, especially the ultimate tensile strength and the elongation at break, are preferably determined by the method disclosed in section "Rheological properties" of the example section (according to the DIN EN ISO 527-1 guideline (Plastics - Determination of tensile properties) and (by supplementation) the USP general chapter <881>). The dissolution time/properties are preferably performed by the method disclosed in the section "Dissolution properties" in the example section (according the method described in Ph. Eur. Chapter 2.9.3).

### Components of the ODF

Generally, the present invention is related to mucosal dissolving films having Buprenorphine as active agent being distributed in a polymeric matrix, comprising of a combination of at least two water-soluble film forming polymers. The active ingredient Buprenorphine HCl or any other pharmaceutically acceptable form of Buprenorphine is uniformly dispersed as particles within this polymeric matrix. The properties of the orally disintegrating film composition can be modified by the molecular ratios of the different polymers within the formulation. Furthermore, surfactants, plasticizers and or other additives known in the field may be used for the manufacturing process to achieve orally disintegrating films with the desired properties.

### • Active Agent according to the present invention

Buprenorphine or Buprenorphine HCl is a semi-synthetic opioid that is used to treat opioid addiction (substitution therapy) in high dosages (>2 mg). It is also used for the management of moderate to severe, acute pain in non-opioid-tolerant individuals (in a low dosage ∼200 µg), and to control moderate, chronic pain in dosages ranging from 20 -70 µg/hour. Buprenorphine is a potent opioid analgesic with a longer duration of action than morphine. It is obtained from the semi-synthetic opioid alkaloid thebaine and works as an agonist/antagonist-mixture affecting different opioid receptors. For that reason, an opioid receptor antagonist (e.g. naloxone) cannot fully reverse the effects of Buprenorphine.

From the prior art, only sublingual tablets from Buprenorphine are known for example under the trade name Subutex^{®} which comprise the active agent Buprenorphine hydrochloride for drug substitution therapy. The suitability of particularly Buprenorphine for drug substitution therapy had been recognized early on in view of Buprenorphine's very long elimination half-life (reported as approximately 20 to 37 hours), which allows a reduced frequency of administration. As a consequence, drug addicts who participate in drug substitution therapy have to report less frequently to the medical agency or healthcare professional supervising the substitution program. Furthermore, the sublingual absorption of Buprenorphine has the advantage that an abuse of Buprenorphine is less likely to occur. The tablets that are currently on the market in the form of Subutex^{®} preparations are for sublingual administration and typically disintegrate over a time period of five to ten minutes. However, within that time period the drug addict may be able to divert the tablet before subsequently either selling the tablets on the street or isolating the active agents therefrom. In order to reduce and eliminate these problems, the present invention provides oral pharmaceutical dosage forms which comprise the active agent Buprenorphine and which release Buprenorphine instantly after oral, preferably sublingual, administration of the drug. It is understood that if reference is made in the context of this invention to the term "Buprenorphine" this refers to the free base as well as to any pharmaceutically acceptable salt thereof such as the hydrochloride, sulfate, bisulfate, tartrate, nitrate, citrate, bitartrate, phosphate, malate, maleate, hydrobromide, hydroiodide, fumarate, succinate salts and the like. A particularly preferred pharmaceutically acceptable salt of Buprenorphine is Buprenorphine hydrochloride.

The orally disintegrating film composition of the present invention may further contain a second active ingredient, such as an opioid antagonist to prevent the isolation or the abuse of the narcotic drug Buprenorphine. As opioid antagonist preferably Naloxone or a pharmaceutical acceptable salt thereof might be used.

### • Dosages

The film may be prepared to have a specific amount of Buprenorphine, such as a specific amount of a combination of Buprenorphine and Opioid-Antagonist drug. The amount of drug included should be consistent across a number of batches so that the ingestible unit can achieve regulatory compliance. Accordingly, the method of preparing an ingestible unit can include: preparing a composition having the pharmaceutical active agent Buprenorphine at a defined amount; forming the composition into a discrete sheet to have the active agent Buprenorphine at a defined amount; and including the discrete sheet in a package. The method may also include: determining a dose of a biologically active agent Buprenorphine to be included in a film.

The amount of Buprenorphine per unit area is determined by the uniform distribution of the film. For example, when the films are cut into individual dosage forms, the amount of the pharmaceutical active agent Buprenorphine in the dosage form can be accurately defined. This is achieved because the amount of Buprenorphine in a given area is substantially identical to the amount of Buprenorphine in an area of the same dimensions in another part of the film. The accuracy in dosage is particularly advantageous for the application of narcotic drug, such as Buprenorphine.

Drug loading into films may be up to about 65% by weight of the film, and often up to about 1% to 20% by dry weight. The amount of drug in each film strip can be calculated before or after shaping the individual films or ingestible unit into the size and shape of the dosage form. The amount of drug lost during processing is usually taken into account in order to design the ingestible units and select the appropriate number of films to arrive at the predetermined dose.

### • Film Forming Polymers

The polymeric matrix of the orally disintegrating film composition is formed by a combination at least two bio-edible and water-soluble or water-swellable polymers.

One of the film-forming polymers is used to predetermine the mechanical and physical properties of the orally disintegrating film strip. This film-forming polymer is selected from the group of starch. The source of the starch may vary and include acorn, arrowroot, arracacha, bananas, barley, beans, breadfruit, buckwheat, canna, cassava, chestnut, chickpea, corn, favas, katakuri, kudzu, lentil, malanga, millet, oat, oca, pea, potato, rice, rye, sago, sorghum, sweet potato, taro, tapioca, water chestnut, wheat, yams and combinations thereof. These starches can be gelatinized, unmodified or modified, including chemically and synthetically modified starch, selected for example from the group of acid-treated starch, alkaline-treated starch, bleached starch, oxidized starch, enzyme-treated starch, monostarch phosphate, distarch phosphate, phosphated distarch phosphate, acetylated distarch phosphate, starch acetate, acetylated distarch adipate, hydroxyethyl starch, hydroxypropyl starch, hydroxypropyl distarch phosphate, hydroxypropyl distarch glycerol, starch sodium octenyl succinate, acetylated oxidized starch.

The second film-forming agent, such as a film-forming polymer, is used to vary the chemical and physical properties of the disintegrating film strip, defined through the properties of the first polymer. By this combination of two film-forming polymers, orally disintegrating films with the desired properties can be achieved. The second film-forming polymer preferably includes pullulan or hydroxypropyl methylcellulose (HPMC). Alternatively, the film may be formed of other synthetic or natural polymers or thickening agents, such as acetylated distarch adipate, agar, alginic acid, beta-glucan, calcium alginate, carrageenan, cassia gum, chondrin, collagen, dextrins, disodium phosphate, disodium pyrophosphate, file powder, galactomannan, gelatin, gellan gum, glucans, glucomannan, guar gum, gulaman, gum karaya, hypromellose, Irvingia gabonensis, konjac, kudzu, bean gum, maltodextrin, methyl cellulose, millet jelly, Monodora pyristica, monosodium phosphate, natural gum, pectin, phosphate; polydextrose, potassium bitartrate, Psyllium seed husk, salep, flour, sodium phosphate, tetrasodium pyrophosphate, tragacanth, waxy corn, methylcellulose, ethylcellulose, hydroxypropylmethyl cellulose, hydroxypropyl cellulose, sodium carboxymethyl cellulose, hydroxyethylcelullose, cellulose acetate phthalate, hydroxypropyl methylcellulose phthalate, carboxymethyl ethylcellulose, hydroxypropylmethyl cellulose acetate succinate, polyvinyl acetate phthalate, sodium alginate, poly (methacrylic acid-co-ethyl acrylate), poly (methacrylic acid co-methyl methacrylate), polyethyleneoxides, polyethyleneglycols, polyvinylpyrrolidone (PVP), polylactic acid (PI-A), poly-L-lactide (PL-LA), poly-D-lactide (PL-DA), poly (lactic co-glycolic acid (PL-GA), chitosan, chitin, xanthan gum, glycogen, poly-HEMA, pOEGMA, microcrystalline cellulose, derivatives thereof and combinations thereof.

In certain embodiments, combined content of all film-forming agents is used in a range of about 20-80 wt%, for example 40-60 wt%, such as about 50 wt%.

In certain embodiment the ratio of the first film-forming polymer to second water-soluble polymer is between 10:1 to about 1:10, preferably between 6:1 to about 1:4, more preferably between 4:1 to about 2:3.

### • Surfactant

For the formation of a stable dispersion of Buprenorphine at least one surfactant and/or cosurfactant may be used. The surfactant may include ionic surfactants including anionic and/or cationic surfactants such as sodium dodecyl sulfate (SDS), sodium lauryl sulfate (SLS), benzalkonium chloride, benzthonium chloride, benzyldimethyldodecyl ammonium bromide (BDDAB), and non-ionic surfactants such as polysorbate 80, sorbitan monooleate, lecithins, glycolipids, fatty alcohols, fatty acids, esters of fatty alcohols and fatty acids, sorbitan esters, polyols such as polysorbates, sorbitans, long-chain aliphatic acids that can be saturated or unsaturated and having more than 6 carbons, such as stearic acid, lauric acid, oleic acid, lineoleic acid, sodium deoxycholate, poloxamers, bile salts such as sodium taurocholate, phospholipids, phosphatidylcholines, phosphatidylethanolamines, phosphatidylinositols, phosphatidylserines, phosphatidic acids, polyglycolized glycerides, polyoxyethylene glycerides, polyethylene glycol-fatty acid esters, polyethylene glycol glycerol fatty acid esters, transesterfication products of alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters derivatives thereof, and combinations thereof. Other suitable surfactants could also be used, and preferably, low molecular weight surfactants are advantageous.

The amounts used of such surfactants can range from about 0.5 wt% to about 20 wt%, and more preferably between 1 wt% to about 10 wt% of total, for example, 4 wt% to 8 wt% of total, although it is possible to use surfactants that are out of this range.

### • Plasticizers

A plasticizer may be used in the formulation of the oral disintegrating film strip. The plasticizer should be a food-grade or pharmaceutical-grade compound, for example one or more of the include low molecular weight polyols such as glycerol, propylene glycol, polyethylene glycols, monosaccharides such as xylitol, erythritol, mannitol, sorbitol; disaccharides such as sucrose, lactose, and maltose; oligosaccharides such as glycogen, inulin, and dextrins such as maltodextrin and similar compounds; citrate derivatives such as citric acid, citrate, tributyl citrate, triethyl citrate, acetyl citrate, citrate ester, triacetin, myglyol, derivatives thereof, and combinations thereof. The amounts used of such plasticizer can range from about 0.5 wt% to about 25 wt%, and more preferably between 5 wt% to about 20 wt% of total, for example, 10 wt% to 15 wt% of total, although it is possible to use plasticizers that are out of this.

### • Coloring/Flavoring/ Sweetening/ Taste masking/ Stabilizing and Thickening Agents

Coloring agents may also be present in the oral disintegrating film and could include titanium dioxide, and dyes suitable for food such as those known as F.D. & C. dyes and natural coloring agents such as grape skin extract, beet red powder, beta-carotene, annatto, carmine, turmeric, paprika, etc. The colorings agent, if present in the film, may range from about 0 to about 2.5 weight percent of the total composition, for example, 1 wt% of the total composition. Flavoring agents may also be present in the oral disintegrating film and could include synthetic flavoring aromatics and/or natural extracts from plants, leaves, flowers, fruits and so forth and combinations thereof. These may include flavors as for example vanilla, citrus flavor, including lemon, orange, grape, lime and grapefruit, and fruit essences, including apple, pear, peach, strawberry, raspberry, cherry, plum, pineapple, apricot and so forth. Flavors which have been found to be particularly useful include commercially available orange, grape, cherry and bubble gum flavors and mixtures thereof. Flavor acids, such as citric acid, malic acid, tartaric acid, lactic acid, and ascorbic acid, may also be used to provide acidic tastes. Flavors may be present in an amount ranging from about 0.5% to about 10.0% by weight based upon the weight of the composition.

Sweetening or taste masking agents may also be present in the oral disintegrating film and could include natural sweeteners, for example, sucrose, stevia, corn syrup, honey, maple syrup, erythritol, maltitol, mannitol, dextrose, fructose, glucose, xylitol, sorbitol, isomalt, and the like, or combinations thereof; and artificial sweeteners, for example, aspartame, sucralose, acesulfame potassium, saccharin, saccharin cyclamate, kleptose, cyclodextrin, cyclodextrin derivatives, and the like, or combinations thereof. The amount of the one or more sweetener agents may range from 0 to 2.5 weight percent, for example, about 1 wt% based on the total weight of the composition.

The oral disintegrating film strip may also include one or more of a permeation or permeability enhancer, a bitter blocker, a filler, an effervescent agent, an anti-oxidant, a disintegrating agent, a pH modifying agent, a buffer, a complexing agent, a bioadhesive, a sheet adhesive, an emulsifying agent, a crystallization inhibitor, a preservative, a unique identifying agent such as a UV active fluorophore, and an antimicrobial.

An anti-oxidant may also be added to the film to prevent the degradation of Buprenorphine, especially where the Buprenorphine form is oxygen- or photosensitive.

### Preparation method of the ODFs

### • Description of formation of the coating mass, homogenization, casting, drying

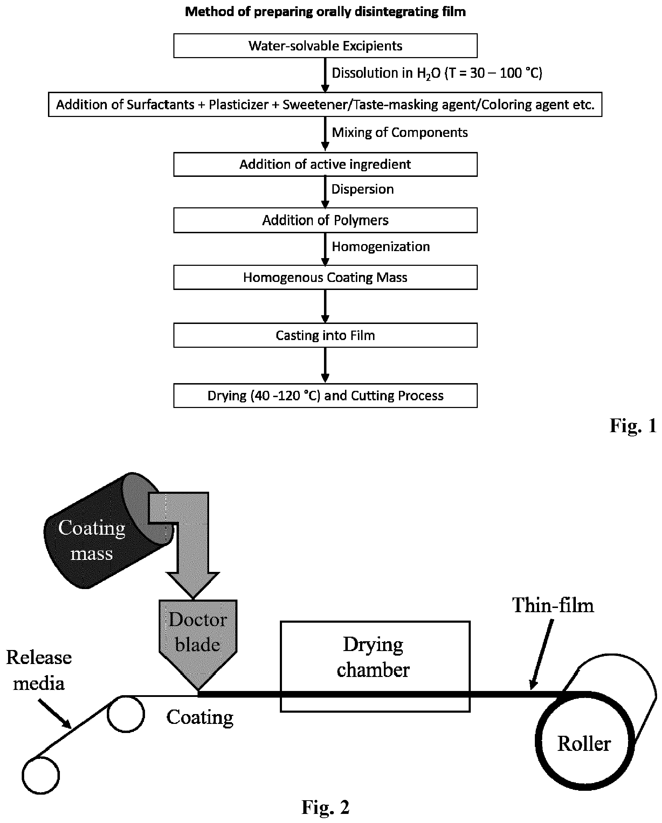

The general procedure for the manufacturing of the orally disintegrating film composition is schematically illustrated in Figure 1.

The water-soluble excipients, like for example water soluble coloring, flavoring, sweetening, taste masking and/or stabilizing agent were dissolved in demineralized water by stirring at 25-90 °C. Subsequently, the surfactants, the plasticizers and Buprenorphine (either as powder form or dissolved or dispersed in a solvent) are added to the mixture and the dispersion is stirred at 25-90 °C. After that, the hydrophilic polymers are added and the dispersion is homogenized at 25-90 °C either by stirring with high rotation speeds (300-1500 rpm) or by mechanical agitation at high shear rates for example by using an Ultra-Turrax^{®} or by applying ultrasonication to the dispersion. Optionally, the resulting dispersion is degassed by stirring or standing at room temperature for (1-24 h) or by stirring briefly under reduced pressure. After that, a sufficient amount of water is added to exactly adjust the desired solid content (20-60 wt% w/w) and viscosity (100-10000 cP) of the final coating mass.

The stable dispersion was then preferably cast to form an oral disintegrating film. The oral disintegrating film was formed by placing the stable dispersion onto a clean receiving substrate or the like to form the oral disintegrating film. The stable dispersion may be dispensed from a suitable apparatus equipped with a doctor blade as would be known in the art, such as a dispensing apparatus, onto the receiving substrate. The stable dispersion may be poured, injected, or otherwise deposited onto the receiving surface by any suitable process or means. The receiving surface can be of any suitable type, such as a tray on a conveyor belt, or a conveyor belt itself. Otherwise, the receiving substrate/surface can be provided in the process e.g. as a continuously moving surface in the form of a release liner which can be easily removed in the course of the further process. The release liner itself can also be constantly moved by a drive roller to guarantee its continuous motion during the casting process (Figure 2).

The cast stable dispersion was dried by being subjected to preferably an infra-red heater system or alternatively heat or hot air in order to form the oral disintegrating films. The apparatus can move the receiving surface through e.g., a convention style oven, or alternatively, the drying can be done in a batch process. In other embodiments, the drying was done with a substrate temperature set to 40-110 °C, for example to about 80 °C, with an infrared-heater system. In a typical embodiment, drying was performed for between about 1 and 15 minutes. After the oral disintegrating film has been dried, it would have a uniform thickness in the range of 100 to 800 microns. The film can be cut into any desirable shape and size, e.g. by a knife/scalpel or by a LASER cutting process. Alternatively, the stable dispersion may be cast into forms of the desired size and shape, which eliminates the cutting step.

One skilled in the art will appreciate that, for this and other processes and methods disclosed herein, the functions performed in the processes and methods may be implemented in differing order. Furthermore, the outlined steps and operations are only provided as examples, and some of the steps and operations may be optional, combined into fewer steps and operations, or expanded into additional steps and operations without detracting from the essence of the disclosed embodiments.

In one embodiment, the oral disintegrating film made with the method of the present invention and Buprenorphine, e.g. Buprenorphine HCl, was found to have percent relative standard deviation of Buprenorphine concentration per strip of less than 2.5%, preferably less than 1.0% and more preferably less than 0.5%. This is as compared to prior art methods, where, in the absence of the creation of a stable dispersion during the process, distribution of Buprenorphine can be much less uniform in the final product.

In certain embodiments, it is advantageous to have a single layer film strip, as described above. A single layer strip typically has a quicker absorption profile. However, in certain other embodiments, a multi-layer film strip is advantageous. A multi-layer film strip may be advantageous if it is desired to keep one pharmaceutically active ingredient away from a second pharmaceutically active ingredient, for example, where the ingredients may interact or degrade. In such an example, a three-layer laminate may be utilized, with an inert layer sandwiched between the two active layers. In other embodiments, it may be advantageous to manufacture a multi-layer film strip having a single active pharmaceutical layer, manufactured as described above, overtop of a mucoadhesive layer, which aids in the adhering of the strip to the oral lingual, sublingual or buccal mucosa of the patient. A multi-layer film strip may also comprise an abrasive layer for enhancing mucosal, sublingual, or buccal delivery, or permeation enhancers.

According to a specifically preferred aspect, the present invention relates to an orally disintegrating film composition comprising Buprenorphine as active ingredient and a polymeric matrix, which is comprising of a combination of at least two water-soluble film forming polymers to form a polymeric matrix and wherein Buprenorphine is uniformly dispersed as particles within the polymeric matrix and wherein the at least two water-soluble film forming polymers are a pregelatinized hydroxypropyl pea starch and pullulan.

According to a preferred embodiment, this specific orally disintegrating film composition has a ratio of pregelatinized hydroxypropyl pea starch and pullulan from 60:40 to 80:20, preferably from 65:35 to 75:25, especially from 67:33 to 73:27.

According to a preferred embodiment, the orally disintegrating film composition has a disintegration time of 120 s or less, preferably of 90 s or less, especially of 60 s or less.

According to a preferred embodiment, the orally disintegrating film composition has an ultimate tensile strength of 0.5 N/mm² or below, preferably of 0.4 N/mm² or below, especially of 0.3 N/mm² or below.

According to another aspect, the present invention also relates to a method for producing an orally disintegrating film composition according to the present invention, wherein a preparation comprising Buprenorphine is mixed with a preparation of the first film-forming polymer, preferably a starch, and a preparation of the second film-forming polymer, preferably pullulan, by stirring with at least 100 rpm for at least 10 min at a temperature of at least 40°C.

Preferably, stirring is performed with at least 200 rpm for at least 30 min at a temperature of 40°C to 65°C, preferably of 45°C to 55°C.

Preferably, the mixture is degassed after mixing for at least 6 h, preferably for at least 10 h.

According to a preferred embodiment, the degassed mixture is adjusted to the desired solid content by adding an aqueous medium, preferably demineralized water.

Preferably, the mixture is casted and dried at a temperature of at least 60°C, preferably at a temperature of 65°C to 100°C.

According to a preferred embodiment, the mixture is casted and dried to a film with a thickness of 10 to 800 µm, preferably of 100 to 500 µm, especially of 130 to 200 µm.

### Examples

Specific ranges, values, and embodiments provided in the examples below are for illustration purposes only and do not otherwise limit the scope of the invention, as defined by the claims. The specific ranges, values, and embodiments described below encompass all combinations and sub-combinations of each disclosed range, value, and embodiment, whether or not expressly described as such. The invention will now be explained more specifically with reference to the following examples and the figures, which are given for illustration of the invention and are not intended to be limiting thereof.

### General Procedure for making orally disintegrating thin films

The general procedure for the manufacturing of the orally disintegrating film composition is schematically illustrated in Figure 1. In a reaction vessel equipped with an overhead stirrer, the water-soluble excipients, e.g. citric acid and sodium citrate, were dissolved in demineralized water by stirring at a minimum of 30 °C for 10-30 min. Subsequently, the surfactants, e.g. Lipoid S 75, the other excipients, e.g. glycerol and sodium saccharin, and if applicable Buprenorphine were added and the mixture was stirred with 300-700 rpm at a minimum of 30 °C for 20-60 min. After that, the hydrophilic polymers, e.g. modified starch and pullulan in the respective ratios, are added and the dispersion was homogenized for a minimum of 30 minutes either by stirring with high rotation speeds (400-1500 rpm) or by mechanical agitation at high shear rates for example by using an Ultra-Turrax^{®} or by applying ultrasonication to the dispersion. Optionally, the resulting dispersion is degassed by stirring or standing at room temperature for (1-24 h) or by stirring briefly under reduced pressure. After that, a sufficient amount of water is added to exactly adjust the desired solid content (20-60 wt% w/w) and viscosity (100-10000 cP) of the final coating mass.

Using a mechanical casting apparatus equipped with doctor blade assembly, the stable coating mass is cast onto a PET substrate at the rate of 0.10-1.0 m/min, followed by drying at 50-90 °C using an IR-drying apparatus (Figure 2) or a convection heating chamber. With this drying process sufficient amounts of water in the composition are removed to produce a uniform thin film having an approximate thickness in the range of 30 to 500 microns. The film was subsequently cut into film strips of the desired size and shape either by carefully cutting the laminates with a knife/scalpel or a punching machine or by using a LASER cutting process. Using the above described ingredients in variable ranges, the manufacturing of an oral disintegrating film using the above described general method involves the formation of a stable wet dispersion, which after casting onto a substrate, produces a thin, semi-opaque film strip with improved mechanical and chemical properties depending on the mixing ratio of the polymers. Thus, the disintegration time or the tensile strength of the orally disintegrating film can be adjusted to produce films with improved properties compared to prior art.

For example, for the composition described in the examples below the disintegration time can be varied in a range from 0.88 min to 2.52 min and the ultimate tensile strength from 0.32 to 0.55 N/mm². If an active ingredient, e.g. Buprenorphine HCl, is included in the orally disintegrating film (Example 5) this general manufacturing procedure ensures a very uniform distribution of Buprenorphine across the film surface, because Buprenorphine is uniformly distributed within the polymeric matrix. Thus, the thin film will have a low relative standard deviation (RSD) of the concentration of Buprenorphine, resulting in more accurate dosing as compared to film strips of the prior art.

### Example 1: Compositions and method of making oral disintegrating films with a ratio of Lycoat:Pullulan of 80:20 without Buprenorphine

In a 500 mL reaction vessel equipped with an overhead stirrer, 17.9 g citric acid and 8.00 g sodium citrate were dissolved in 253.8 g demineralized water by stirring with 300 rpm at 30 °C for 10 min. Subsequently, 1.32 g Lipoid S 75, 21.6 g Glycerol and 1.26 g sodium saccharin were added and the mixture was stirred with 500 rpm at 30 °C for 20 min. After that, 60.0 g Lycoat NG 720 and 15.0 g Pullulan are added and the dispersion was homogenized by stirring with 400 rpm at 50 °C for ca. 2h. The resulting dispersion was degassed by stirring with 200 rpm at 50 °C for 15 min and by standing at room temperature for 18 h. The formulation of the Examples 1 is summarized in Table 1. After that, water was added to achieve the desired solid content (ca. 32 - 33 wt%) of the final coating mass. Using a mechanical casting apparatus equipped with doctor blade assembly and convection heating chamber, the stable dispersion is cast onto a PET substrate at the rate of 0.12 m/min and a gap width of 280 µm, followed by drying at 75-90 °C using an IR-drying apparatus. With this drying a uniform thin film having a thickness of about 87 µm and a dry coating weight of 12.2 mg/cm² was produced.

**Table 1. Compositions of the orally disintegrating thin film with a ratio for Lycoat:Pullulan of 80:20.**

| Example 1 | |
|---|---|
| Component | wt% in dry mass |
| Lycoat NG 720 | 48.0 |
| Pullulan | 12.0 |
| Glycerol | 17.3 |
| Lipoid S75 | 1.0 |
| Citric Acid | 14.3 |
| Sodium Citrate | 6.4 |
| Saccharin-Sodium | 1.0 |
| | |
| Total | 100 |

### Example 2, 3 and 4: Oral disintegrating films with different ratios of starch and pullulan without Buprenorphine

Examples 2, 3 and 4 are compositions of orally disintegrating films prepared by using the same method as in Example 1, with different ratios of the film-forming polymers Lycoat NG 720 and pullulan to modify the physical and chemical properties of the films. The following ratios of Lycoat:pullulan were used without changing any other quantity or component of the composition: 60:40 (Example 2), 40:60 (Example 3) and 20:80 (Example 4). The detailed formulations of the examples 2, 3 and 4 are summarized in Table 2.

The coating process of these Examples was performed analogous to the manufacturing process of Example 1.

**Table 2. Compositions of the orally disintegrating thin films with modified ratios of the film-forming polymers starch:pullulan (Example 2: 60:40, Example 3: 40:60 and Example 4: 20:80).**

| Example 2 | | Example 3 | | Example 4 | |
|---|---|---|---|---|---|
| Component | wt% in dry mass | Component | wt% in dry mass | Component | wt% in dry mass |
| Lycoat NG720 | 36.0 | Lycoat NG720 | 24.0 | Lycoat NG720 | 12.0 |
| Pullulan | 24.0 | Pullulan | 36.0 | Pullulan | 48.0 |
| Glycerol | 17.3 | Glycerol | 17.3 | Glycerol | 17.3 |
| Lipoid S75 | 1.0 | Lipoid S75 | 1.0 | Lipoid S75 | 1.0 |
| Citric Acid | 14.3 | Citric Acid | 14.3 | Citric Acid | 14.3 |
| Sodium Citrate | 6.4 | Sodium Citrate | 6.4 | Sodium Citrate | 6.4 |
| Saccharin-Sodium | 1.0 | Saccharin-Sodium | 1.0 | Saccharin-Sodium | 1.0 |
| | | | | | |
| Total | 100 | Total | 100 | Total | 100 |

### Example 5: Example of an orally disintegrating film containing Buprenorphine HCl

In Example 5 Buprenorphine HCl is incorporated as active pharmaceutical ingredient in the orally disintegrating film. This example demonstrates that Buprenorphine can easily be incorporated and uniformly distributed in the orally disintegrating film composition of the invention. The complete composition of Example 5 is summarized in Table 3. The orally disintegrating film of Example 5 was prepared according to the general procedure described above.

In a reaction vessel equipped with an overhead stirrer, 31.1 g citric acid and 14.1 g sodium citrate dihydrate were dissolved in 443 g demineralized water by stirring with 300 rpm at 30 °C for 10 min. Subsequently, 2.1 g Lipoid S 75, 37.5 g glycerol, 2.0 g sodium saccharin and 45.5 g Buprenorphine HCl were added and the reaction mixture was stirred with 500 rpm at 30 °C for 25 min. After that, 37.8 g Pullulan and 80.2 g Lycoat^{®} NG 720 were added and the dispersion was homogenized by stirring with 500 rpm at 50 °C for 80 min. The rotation speed was reduced to 200 rpm and the reaction mixtures was stirred at 50 °C for 35 min. The resulting mixture was degassed by standing at room temperature for 18 h. After that, a 63 g of demineralized water was added to exactly adjust the desired solid content to 33.5 wt% and the viscosity of the final coating mass.

Using a mechanical casting apparatus equipped with doctor blade assembly the stable dispersion is cast onto a PET substrate at the rate of 0.12 m/min and a gap width of 250 µm, followed by drying at 75-90 °C using an IR-drying apparatus. With this drying a uniform thin film having a uniform thickness of about 165 µm and a dry coating weight of 12.6 mg/cm² were produced.

This Example shows that the active ingredient, Buprenorphine HCl, can easily be incorporated in the composition of the present invention to obtain an orally disintegrating film with the desired properties.

**Table 3. Compositions of the orally disintegrating thin film containing Buprenorphine HCl as active ingredient.**

| Example 5 | |
|---|---|
| Component | wt% |
| Buprenorphine HCl | 18.2 |
| Lycoat NG720 | 32.0 |
| Pullulan | 15.1 |
| Glycerol | 15.0 |
| Lipoid S75 | 0.9 |
| Citric acid | 12.4 |
| Sodium Citrate Dihydrate | 5.6 |
| Saccharin Sodium Dihydrate | 0.8 |
| | |
| Total | 100 |

### Example 6: Example of an orally disintegrating film with no Pullulan and only Lycoat as polymer

The example 6 is a composition of an orally disintegrating film prepared by using the same method as in Example 5, but only Lycoat and no Pullulan was used as polymer. This example was manufactured to compare the mechanical properties of this example with the examples containing a ratio of Lycoat: Pullulan 70:30. The detailed formulation of example 6 is summarized in Table 4. The manufacturing and coating process of this Example was performed analogous to the general manufacturing process of Example 1.

**Table 4. Compositions of the orally disintegrating thin film containing Buprenorphine with Lycoat as only polymer.**

| Example 6 | |
|---|---|
| Component | wt% in dry mass |
| Lycoat NG720 | 80 |
| Pullulan | - |
| Glycerol | 15 |
| Saccharin Sodium | 3.0 |
| Lecithin | 2.0 |
| | |
| Total | 100 |

### Example 7, 8 and 9: Further example of an orally disintegrating film containing Buprenorphine HCl

Examples 7,8 and 9 are compositions of orally disintegrating films prepared by using the same method as in Example 5. In the following examples a Lycoat NG 720:pullulan ratio of 70:30 was used and the mechanical and chemical properties of these examples was compared. The detailed formulations of the examples 7,8 and 9 are summarized in Table 5.

The coating process of these Examples was performed analogous to the general manufacturing process of Example 1.

**Table 5. Compositions of the orally disintegrating thin films containing Buprenorphine with a ratio of the film-forming polymers starch:pullulan of 70:30.**

| Example 7 | | Example 8 | | Example 9 | |
|---|---|---|---|---|---|
| Component | wt% in dry mass | Component | wt% in dry mass | Component | wt% in dry mass |
| Buprenorphine HCl | 20.1 | Buprenorphine HCl | 17.5 | Buprenorphine HCl | 17.5 |
| Lycoat NG720 | 43.7 | Lycoat NG720 | 43.7 | Lycoat NG720 | 33.9 |
| Pullulan | 19.0 | Pullulan | 19.0 | Pullulan | 15.1 |
| Glycerol | 16.2 | Glycerol | 17.7 | Glycerol | 14.7 |
| Saccharin Sodium | 1.0 | Lecithin | 1.1 | Lecithin | 0.8 |
| | | Saccharin Sodium | 1.0 | Citric acid | 11.9 |
| | | | | Sodium Citrate | 5.4 |
| | | | | Saccharin Sodium | 0.8 |
| | | | | | |
| Total | 100 | Total | 100 | Total | 100 |

### Investigation of the properties of ODFs with different ratios of polymers (Examples 1-5)

### • Physical properties

Although all examples were produced according the similar manufacturing procedure for the preparation of the coating mass and for the coating process, the modification of the ratios of the two-hydrophilic polymers in the studied ODF examples provokes a strong variation of the material properties of the ODFs. For example, the dry coating weight (DCW) and the dry coating thickness (DCT) of the investigated ODFs are strongly decreased for higher molar ratios of pullulan concomitant with an increase of their densities (Table 6). Thus, Example 1 exhibits a much higher DCW (12.2 mg/cm²) and DCT (87 µm) compared to Example 2 (10.4 mg/cm², 75 µm), Example 3 (9.12 mg/cm², 66 µm) and Example 4 (8.13 mg/cm², 59 µm). These differences in the medicinal product properties are in good accordance with the measured viscosities, which are mainly influenced by the variation of different polymers, of the different coating masses utilized in the casting process. For a coating mass with lower viscosity a thin film with higher thickness (associated with a higher DCW) is created by applying a constant gap width of the doctor blade during the process, while for a coating mass with high viscosity the thickness of the produced film is lower. The above described behavior illustrates that the product properties of the ODFs can be adjusted by variation of the ratios of the different polymers.

In the formulation of Example 5 Buprenorphine HCl is included as active ingredient in the ODFs in contrast to the Examples 1-4 without Buprenorphine. In Example 5 the density of the thin-films is lower compared to the other examples because of Buprenorphine included in the polymeric matrix as a very bulky solid dispersion. As a consequence of the very low density, the orally disintegrating films of Example 5 exhibit a much higher dry coating thickness to obtain a similar dry coating weight compared to the other examples. Nevertheless, Example 5 shows that Buprenorphine HCl can be uniformly incorporated in the polymeric matrix.

**Table 6. Physical properties of the ODFs with different ratios of starch:pullulan.**

| | Ratio starch:pullulan | Dry coating weight (DCW) [mg/cm²] | Dry coating thickness (DCT) [µm] | Density [g/cm³] |
|---|---|---|---|---|
| Example 1 | 80:20 | 12.2 | 87 | 1.40 |
| Example 2 | 60:40 | 10.4 | 75 | 1.39 |
| Example 3 | 40:60 | 9.12 | 66 | 1.38 |
| Example 4 | 20:80 | 8.13 | 59 | 1.37 |
| Example 5* | 68:32 | 12.5 | 165 | 0.76 |

| | | | | |
|---|---|---|---|---|
| [*] Example 5 contains Buprenorphine HCl in contrast to the Examples 1-4. | | | | |

### • Water content of the ODFs

Although all ODFs were manufactured according to the same manufacturing procedure, the content of water in the orally disintegrating thin-films can be modified by the ratio of the polymers. Therefore, the water content of above described ODF examples was determined by measuring the loss on drying of the ODFs in a conventional vacuum drying cabinet. The loss on drying is determined with a method based on the Chapter *loss on drying* (Ph. Eur. 2.2.32). A sample of about 1.00 g of the substance is accurately balanced in weighing vessel (predried) and placed in a vacuum drying oven. The sample is dried to constant weight at 105 °C ± 2 °C and cooled down to room temperature in a desiccator. The weighing vessel is balanced again until the difference between two subsidiary weights is below 0.5 mg/g and the ratio of volatile content is calculated. Since glycerol may also evaporate for very long heating times, the loss on drying after 1h at 105 °C was considered for a comparison of the actual water content of the ODFs. The results are summarized in Table 7.

This study revealed that the water content of the ODFs in equilibrium conditions is strongly dependent on the molecular ratio of the polymers, although the drying procedure and temperature of the all examples was constant. For a higher ratio of the hydrophilic Lycoat more water can be incorporated into the polymeric matrix leading to a higher water content in this ODFs. Thus, Example 1 shows with the highest ratio of Lycoat:Pullulan (80:20) the highest water content of 8.7% in the ODFs compared to Example 2 (7.7%), Example 3 (7.0%) and Example 4 (6.4%). This relationship can be utilized to produce ODFs with distinct contents of water by modification of the ratio of polymers.

Since in the formulation of Example 5 Buprenorphine HCl is included as active ingredient in the ODFs in contrast to the Examples 1-4, the loss on drying of the orally disintegrating films of Example 5 is higher than that of the other examples of the series without Buprenorphine.

**Table 7. Loss on drying of the ODFs with different ratios of Lycoat:pullulan.**

| | Ratio starch:pullulan | water content [wt%] |
|---|---|---|
| Example 1 | 80:20 | 8.7 |
| Example 2 | 60:40 | 7.7 |
| Example 3 | 40:60 | 7.0 |
| Example 4 | 20:80 | 6.4 |
| Example 5 | 68:32 | 9.9 |

| | | |
|---|---|---|
| [*] Example 5 contains Buprenorphine HCl in contrast to the Examples 1-4. | | |

### • Disintegration

The disintegration of the thin films was *in vitro* characterized in a solution of phosphate buffered saline (pH = 6.8) at 25 °C to mimic the oral cavity. For the preparation of the dissolution medium 8 g of sodium chloride, 0.2 g of potassium dihydrogen orthophosphate and 2.66 g of disodium hydrogen phosphate dihydrate are dissolved into 1000 mL of demin. water and the pH is adjusted to 6.8 by addition of ortho-phosphoric acid (85%). 25 mL of buffer are filled into a petri dish and are placed onto an orbital shaker. The oral thin film is placed into the petri dish by using a tweezer and the samples are swiveled at a specific rate (75 rpm) to stimulate movement in the solution. The time for complete disintegration is recorded for 6 replicates of each oral thin film example. The reported disintegration times are the averages of the replicate measurements and are summarized in the following Table 8.

**Table 8. Disintegration times of the ODFs with different ratios of starch:pullulan.**

| | Ratio starch:pullulan | Disintegration time [min] |
|---|---|---|
| Example 1 | 80:20 | 0.88 |
| Example 2 | 60:40 | 1.00 |
| Example 3 | 40:60 | 1.15 |
| Example 4 | 20:80 | 2.52 |
| Example 5* | 68:32 | 1:23 |

| | | |
|---|---|---|
| [*] Example 5 contains Buprenorphine HCl as active ingredient in contrast to the Examples 1-4. | | |

These results indicate that changing the ratio of the hydrophilic polymers within the ODFs drastically influence the properties of the ODFs. An high starch:pullulan ratio (Example 1), leads to much faster disintegration (0.88 min) compared to an ODF with a smaller amount of starch (Example 2, 1.00), while a further decrease of the ratio (Example 3 and Example 4) leads to slower disintegration times (1.15 min and 2.52 min). The use of a higher amount of pullulan leads to a higher stability of the ODFs concomitant with an increase of the disintegration time, because pullulan does stabilize the polymer matrix. This very strong effect of the polymeric composition of the ODF is even more striking, since the dry coating weight and dry coating thickness of the ODFs might have an opposing effect on the disintegration. Although Example 4 has the lowest dry coating weight and thickness of the present series, which should lead to faster disintegration, it actually showed the slowest disintegration (2.52 min) of the series. This behavior illustrates that the variation of the polymeric ratio strongly influences the properties of the polymeric matrix of the ODFs and compensates the effect of the weight and size of the thin-films. The study of the disintegration revealed that the material properties of the ODFs can be gravely varied by the ratio of polymeric components of the formulation of the orally disintegrating film.

Although in Example 5 Buprenorphine HCl is included in the formulation, the disintegration time is in the same range as for the Example without Buprenorphine (Example 1-4). This results further corroborate that the disintegration properties are mainly determined by polymers.

### • Rheological properties

The rheological properties were determined according to the DIN EN ISO 527-1 guideline (*Plastics* - *Determination of tensile properties*) and the USP general chapter <881>. For the determination of the ultimate tensile strength and the elongation at break of the different thin film formulations, bone-shape samples with a length of 115 mm and measurement cross-section of 6 ± 0.4 mm were cut out of the laminates of the ODFs. The samples were investigated with the tension meter T300 with a velocity of 5 mm/min at 23 °C and 50% RH. From these experiments the ultimate tensile strength and the elongation at break can be determined. The ultimate tensile strength, which is the maximum force applied until the breakage of the ODFs, is calculated by dividing the maximal force (N) the material can withstand through the cross-sectional area (mm²). The elongation at break (%) is the elongation at the time point when the sample starts to break. The values of the elongation at break and the ultimate tensile strength are summarized in Table 9. The variation of the ratio of the polymers has a huge impact on the elasticity and stiffness of the material of the ODFs. For a higher pullulan content more ductile and elastic films can be produced. Accordingly, the elasticity determined for Example 1 (elongation at break: 189%) is much lower than that of the other Examples (elongation at break: > 240%).

This trend is also reflected in the in the tendency of the ultimate tensile strength, which is the maximum stress that the material can withstand while being stretched before breaking, of the investigated films. The studies of the ultimate tensile strength illustrate that the films with a higher pullulan content are more robust to applied stress than those with lower pullulan content. Consequently, Example 4 has the highest ultimate tensile strength (0.55 N/mm²) compared to Example 3 (0.51 N/mm²), Example 2 (0.41 N/mm²) and Example 1 (0.32 N/mm²). These results illustrate, that by including a higher ratio of the more flexible polymer (Pullulan), ODFs with a higher flexibility and lower stiffness are produced compared to the examples with a higher ratio of Lycoat.

These results can be confirmed by a comparison with Example 6. In this Example Lycoat is used as a single polymer without the beneficial combination with Pullulan. The elasticity of this example is drastically lowered (elongation break: 18% and ultimate tensile 20.8 N/mm²) and the films of this example are very brittle. Therefore, these films break very easily during the manufacturing process and cannot be applied as orally disintegrating films. This example illustrates clearly that a combination of polymers within an orally disintegrating film is necessary to obtain process able films and to adapt the mechanical properties of the orally disintegrating films. This study confirms that the physical properties of the orally disintegrating film compositions are strongly determined by the molecular properties and the molecular ratios of the used polymers.

**Table 9. Rheological properties of ODFs with different polymer ratios of starch:pullulan.**

| | Ratio starch:pullulan | Elongation at break [%] | Ultimate Tensile strength [N/mm²] |
|---|---|---|---|
| Example 1 | 80:20 | 189 | 0.32 |
| Example 2 | 60:40 | > 240 | 0.41 |
| Example 3 | 40:60 | > 240 | 0.51 |
| Example 4 | 20:80 | > 240 | 0.55 |
| Example 6 | 100:0 | 18 | 20.8 |

### Investigation of the properties of ODFs containing Buprenorphine in comparison to the sublingual tablet Subutex^{®} (Examples 5, 7, 8 and 9)

### • Disintegration

The disintegration of the thin films was *in vitro* characterized in a solution of phosphate buffered saline (pH = 6.8) at 25 °C to mimic the oral cavity. 25 mL of buffer are filled into a petri dish and are placed onto an orbital shaker. The oral thin film is placed into the petri dish by using a tweezer and the samples are swiveled at a specific rate (75 rpm) to stimulate movement in the solution. The disintegration of the sublingual tablets of Subutex ^{®} with two dosages (2 and 8 mg) was determined with the same method described before. The determined disintegration times of the examples 5, 7, 8 and 9 are depicted in the following Table 10 compared to the disintegration of Subutex^{®}.

**Table 10. Comparison of the disintegration times of the ODFs of the Examples 5, 7, 8 and 9 with the sublingual tablets Subutex^{®}.**

| | Disintegration time [min] |
|---|---|
| Example 5 | 1:23 |
| Example 7 | 0:48 |
| Example 8 | 0:23 |
| Example 9 | 1:40 |
| Subutex^{®} 2 mg | 3:35 |
| Subutex^{®} 8 mg | 16:07 |

Example 8 (23 s) shows a faster disintegration than Example 7 (48 s), Example 5 (1:23 min) and Example 9 (1:40 min), but the disintegration of all the examples of the present invention containing Buprenorphine is much faster than the disintegration of the sublingual tablets (3:35 min containing 2 mg of Buprenorphine and 16:07 with 8 mg Buprenorphine). These results illustrate that in regard to disintegration the ODFs of the present invention are superior compared to the reference formulation. The faster disintegration of the orally disintegrating films of the present invention allows a faster and more efficient release of the Buprenorphine. The feature of the faster release of the active ingredient can also be observed in the dissolution profile of the orally disintegrating films (see below).

### • Dissolution properties

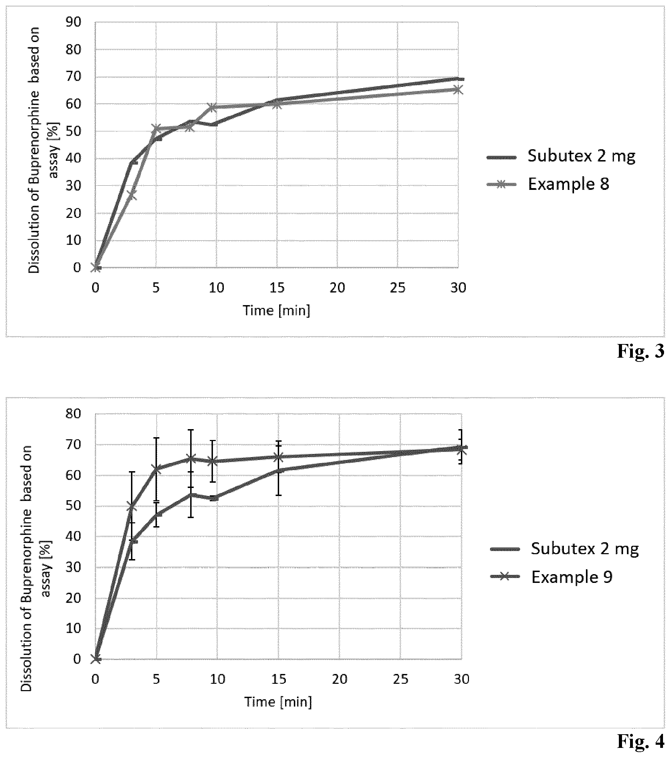

The dissolution of the orally disintegrating films of the Examples 8 and 9 were investigated in a saliva buffer with pH of 6.8 based on the method described in Ph. Eur. Chapter 2.9.3. For the preparation of the dissolution medium 32 g of sodium chloride, 0.8 g of potassium dihydrogen orthophosphate and 10.6 g of disodium hydrogen phosphate dihydrate are dissolved into 4000 mL of demin. water and the pH is adjusted to 6.8 by addition of ortho-phosphoric acid (85%). The dissolution of the ODFs is determined with a rotating basket apparatus at a temperature of 37 ± 0.5 °C and 100 cycles per minute. During the dissolution experiment samples (1.0 mL) were taken from the dissolution vessel (500 mL) after 3, 5, 8, 10, 15 and 30 min. The content of Buprenorphine in these samples is analyzed by HPLC. The results of the dissolution experiments of ODFs containing 2 mg of Buprenorphine were compared to Subutex^{®} 2 mg and are shown in Table 11 and the Figures 3 and 4, respectively.

The studies indicate that the similarity between the reference formulation Subutex^{®} and the developed ODF examples of the present invention. In all cases a dissolution of around 60-70% is reached at the endpoint of the dissolution measurement after 30 minutes. It was demonstrated that the dissolution of the developed formulation was comparable to the reference tablets in the dissolution medium. Moreover, in Example 8 the dissolution is distinctly faster than the dissolution of the reference indicating the beneficial properties of the present invention for the dissolution of Buprenorphine.

**Table 11. Results of the dissolution test of Buprenorphine based on the assay of the examples 8 and 9.**

| Sample | 3 min | 5 min | 7.5 min | 10 min | 15 min | 30 min |
|---|---|---|---|---|---|---|
| Example 8 [%] | 26.7 | 50.9 | 51.6 | 58.7 | 59.9 | 65.3 |
| Example 9 [%] | 49.8 | 61.9 | 65.4 | 66.0 | 62.3 | 68.9 |
| Subutex^{®} | 38.4 | 47.0 | 53.6 | 52.4 | 61.5 | 69.2 |

### • Summary of the Examples

In conclusion, the studies of the above described examples, which are manufactured according to the same procedure described in the present invention, demonstrate that the properties of the ODFs can be tuned by variation of the ratio of polymers in the formulation. In the described examples two different hydrophilic polymers were combined to form one thin film composition to obtain disintegrating materials with novel properties, which are not accessible with previous methods or by using only a single film-forming polymer. Moreover, with the manufacturing procedure described in the invention the production of ODFs with precisely adapted and highly desired properties and features can be produced. Finally, Example 5 demonstrates that Buprenorphine HCl as an active ingredient can easily be included within the orally disintegrating films of the present invention and still the properties of the ODFs can be controlled by the utilization of a combination of different polymers to achieve the desired medicinal product.

Therefore, the present invention discloses the following preferred embodiments:
1. The orally disintegrating film composition comprising Buprenorphine as active ingredient and a polymeric matrix, which is comprising of a combination of at least two water-soluble film forming polymers to form a polymeric matrix and wherein Buprenorphine is uniformly dispersed as particles within the polymeric matrix.
2. The orally disintegrating film composition of embodiment 1, wherein the composition has a disintegration time of 180 s or less, preferably of 120 s or less, especially of 90 s or less and/or an ultimate tensile strength of 0.8 N/mm² or below, preferably of 0.5 N/mm² or below, especially of 0.3 N/mm² or below.
3. The orally disintegrating film composition of embodiment 1 or 2, wherein Buprenorphine, is Buprenorphine free base or a pharmaceutically acceptable salt thereof, such as the hydrochloride, sulfate, bisulfate, tartrate, nitrate, citrate, bitartrate, phosphate, malate, maleate, hydrobromide, hydroiodide, fumarate, or succinate; and/or wherein the dosage form additionally comprises an opioid antagonist, preferably naloxone or a pharmaceutical acceptable salt thereof.
4. The orally disintegrating film composition of any one of embodiments 1 to 3, wherein the first film-forming polymer is a starch selected from the group consisting of acorn, arracacha, barley, beans, breadfruit, buckwheat, cassava, chickpea, chestnut, corn, lentil, millet, oat, pea, potato, rice, rye, sago, sorghum, sweet potato, tapioca, wheat and combinations thereof, preferably wherein the starch is a pregelatinized starch, a hydroxypropyl starch or a pregelatinized hydroxypropyl starch, especially a pregelatinized hydroxypropyl pea starch.
5. The orally disintegrating film composition of embodiment 4, wherein said starch is pregelatinized, gelatinized, modified or unmodified.
6. The orally disintegrating film composition of any one of embodiments 1 to 5, wherein the second film forming polymer is selected from the group comprising glucans, pullulan, dextrin, gelatins, glycogen, methylcellulose, ethylcellulose, hydroxypropylmethylcellulose, hydroxyethylcelullose, cellulose acetate phthalate, hydroxypropyl methylcellulose phthalate, carboxymethyl ethylcellulose, hydroxypropylmethyl cellulose acetate succinate, polyvinyl acetate phthalate, maltodextrin, dextran, hydroxypropyl cellulose, sodium carboxymethyl cellulose, poly(methacrylic acid-co-ethyl acrylate), poly(methacrylic acid-co-methyl methacrylate), polyethyleneoxide, polyethyleneglycol, polyvinylpyrrolidone (PVP), polylactic acid (PLA), poly-L-lactide (PLLA), poly-D-lactide (PLDA), poly(lactic-co-glycolic acid) (PLGA), and mixtures thereof, preferably pullulan, more preferred pullulan with a viscosity of 100 mm²/s to 180 mm²/s, especially pullulan with a viscosity of 140 mm²/s to 160 mm²/s.
7. The orally disintegrating film composition of any one of the preceding embodiments, wherein the ratio of the first film-forming polymer to the second water-soluble polymer is about 10:1 to about 1:10, preferably between 6:1 to about 1:4, more preferably between 4:1 to about 2:3.
8. The orally disintegrating film composition of any one of embodiments 1 to 7, wherein a surfactant and/or cosurfactant is used for the homogenous dispersion of Buprenorphine within the polymeric matrix.
9. The orally disintegrating film composition of embodiment 8, wherein the surfactant and/or cosurfactant is selected from the group consisting of polyglycolized glycerides, polyoxyethylene glycerides, polyethylene glycol-fatty acid esters, polyethylene glycol glycerol fatty acid esters, transesterfication products of alcohols, polyglycerized fatty acids, glycerol fatty acid esters, polyglycerol fatty acid esters, propylene glycol fatty acid esters, mono and diglycerides, polyoxyethylene-polyoxypropylene block copolymers, polyethylene glycol sorbitan fatty acid esters, sorbitan fatty acid esters, polysorbates, poloxamers, phospholipids, lecithin, phosphatidylcholines, phosphatidylethanolamines, phosphatidylinositols, phosphatidylserines, phosphatidic acids, and mixtures thereof.
10. The orally disintegrating film composition of any one of the preceding embodiments, wherein the film comprises one or more plasticizers, taste masking agent, a flavoring agent, a solubilizer, a thickener, a coloring agent, an effervescent agent, an antioxidant, a pH modifying agent, vitamins, minerals, a dietary supplement, or mixtures thereof.
11. The orally disintegrating film composition of embodiment 10, wherein the flavoring and/or taste masking agent is present in an amount of up to 10% by weight of the film and is selected from the group comprising any one of kleptose, cyclodextrin, cyclodextrin derivatives, ginger, anise, cinnamon, peppermint, licorice, fruit juice, citric acid, citrate, sweeteners, sucrose, glucose, fructose, mannitol, saccharin, aspartame, sucralose, stevia plant derivatives, honey, or any combination thereof.
12. The orally disintegrating film composition of any one of the preceding embodiments, wherein Buprenorphine is present in amounts of up to about 0.01% to about 50% by weight of the total composition, preferably 1% to about 30%, more preferably 10% to about 20%.
13. The orally disintegrating film composition of any one of the preceding embodiments, wherein the composition is essentially free of a gum resin.
14. The orally disintegrating film composition of any one of the preceding embodiments, wherein the composition consists of a single layer.
15. The orally disintegrating film composition of any one of the preceding embodiments, having a polymeric backing layer.
16. The orally disintegrating film composition of any one of the preceding embodiments, wherein the thickness of the film ranges from about 0.01 to 0.8 mm.
17. The orally disintegrating film composition of any one of the preceding embodiments, wherein the composition is provided without utilizing an oily medium.
18. The orally disintegrating film composition of any one of the preceding embodiments, wherein dissolution time in phosphate buffered saline solution at pH 6.8 and 25 °C is determined by the mixture of two hydrophilic polymers and is less than about 10 minutes, preferably less than about 2 minutes, more preferably less than about 1 minute.
19. The orally disintegrating film composition of any one of the preceding embodiments, wherein the mechanical and physical properties of the film are modified by the variation of the ratio of two hydrophilic polymers within the formulation.
20. The orally disintegrating film composition comprising Buprenorphine as active ingredient and a polymeric matrix, which is comprising of a combination of at least two water-soluble film forming polymers to form a polymeric matrix and wherein Buprenorphine is uniformly dispersed as particles within the polymeric matrix and wherein the at least two water-soluble film forming polymers are a pregelatinized hydroxypropyl pea starch and pullulan.
21. The orally disintegrating film composition according to embodiment 20, wherein the ratio of pregelatinized hydroxypropyl pea starch and pullulan is from 60:40 to 80:20, preferably from 65:35 to 75:25, especially from 67:33 to 73:27.
22. The orally disintegrating film composition of any one of embodiments 1 to 21, wherein the film composition has a disintegration time of 180 s or less, preferably of 120 s or less, especially of 60 s or less.
23. The orally disintegrating film composition of any one of embodiments 1 to 22, wherein the film composition has an ultimate tensile strength of 0.8 N/mm² or below, preferably of 0.5 N/mm² or below, especially of 0.3 N/mm² or below.
24. The orally disintegrating film composition of any one of embodiments 1 to 23, wherein the film composition has an ultimate tensile strength of 0.5 N/mm² or below, especially of 0.3 N/mm² or below.
25. A method for producing an orally disintegrating film composition according to any one of embodiments 1 to 24, wherein a preparation comprising Buprenorphine is mixed with a preparation of the first film-forming polymer, preferably a starch, and a preparation of the second film-forming polymer, preferably pullulan, by stirring with at least 100 rpm for at least 10 min at a temperature of at least 40°C.
26. A method according to embodiment 25, wherein stirring is performed with at least 200 rpm for at least 30 min at a temperature of 40°C to 65°C, preferably of 45°C to 55°C.
27. A method according to embodiment 25 or 26, wherein the mixture is degassed after mixing for at least 6 h, preferably for at least 10 h.
28. A method according to embodiment 27, wherein the degassed mixture is adjusted to the desired solid content by adding an aqueous medium, preferably demineralized water.
29. A method according to any one of embodiments 25 to 28, wherein the mixture is casted and dried at a temperature of at least 60°C, preferably at a temperature of 65°C to 100°C.
30. A method according to any one of embodiments 25 to 29, wherein the mixture is casted and dried to a film with a thickness of 10 to 800 µm, preferably of 100 to 500 µm, especially of 130 to 200 µm.

## Claims

1. The orally disintegrating film composition comprising Buprenorphine as active ingredient and a polymeric matrix, which is comprising of a combination of at least two water-soluble film forming polymers to form a polymeric matrix and wherein Buprenorphine is uniformly dispersed as particles within the polymeric matrix, and wherein the composition has a disintegration time of 180 s or less, preferably of 120 s or less, especially of 90 s or less and/or an ultimate tensile strength of 0.8 N/mm² or below, preferably of 0.5 N/mm² or below, especially of 0.3 N/mm² or below.

2. The orally disintegrating film composition of claim 1, wherein Buprenorphine, is Buprenorphine free base or a pharmaceutically acceptable salt thereof, such as the hydrochloride, sulfate, bisulfate, tartrate, nitrate, citrate, bitartrate, phosphate, malate, maleate, hydrobromide, hydroiodide, fumarate, or succinate; and/or wherein the dosage form additionally comprises an opioid antagonist, preferably naloxone or a pharmaceutical acceptable salt thereof.

3. The orally disintegrating film composition of claim 1 or 2, wherein the first film-forming polymer is a starch selected from the group consisting of acorn, arracacha, barley, beans, breadfruit, buckwheat, cassava, chickpea, chestnut, corn, lentil, millet, oat, pea, potato, rice, rye, sago, sorghum, sweet potato, tapioca, wheat and combinations thereof, preferably wherein the starch is a pregelatinized starch, a hydroxypropyl starch or a pregelatinized hydroxypropyl starch, especially a pregelatinized hydroxypropyl pea starch.

4. The orally disintegrating film composition of claim 3, wherein said starch is pregelatinized, gelatinized, modified or unmodified.

5. The orally disintegrating film composition of any one of claims 1 to 4, wherein the second film forming polymer is selected from the group comprising glucans, pullulan, dextrin, gelatins, glycogen, methylcellulose, ethylcellulose, hydroxypropylmethylcellulose, hydroxyethylcelullose, cellulose acetate phthalate, hydroxypropyl methylcellulose phthalate, carboxymethyl ethylcellulose, hydroxypropylmethyl cellulose acetate succinate, polyvinyl acetate phthalate, maltodextrin, dextran, hydroxypropyl cellulose, sodium carboxymethyl cellulose, poly(methacrylic acid-co-ethyl acrylate), poly(methacrylic acid-co-methyl methacrylate), polyethyleneoxide, polyethyleneglycol, polyvinylpyrrolidone (PVP), polylactic acid (PLA), poly-L-lactide (PLLA), poly-D-lactide (PLDA), poly(lactic-co-glycolic acid) (PLGA), and mixtures thereof, preferably pullulan, more preferred pullulan with a viscosity of 100 mm²/s to 180 mm²/s, especially pullulan with a viscosity of 140 mm²/s to 160 mm²/s.

6. The orally disintegrating film composition of any one of the preceding claims, wherein the ratio of the first film-forming polymer to the second water-soluble polymer is about 10:1 to about 1:10, preferably between 6:1 to about 1:4, more preferably between 4:1 to about 2:3.

7. The orally disintegrating film composition of any one of the preceding claims, wherein Buprenorphine is present in amounts of up to about 0.01% to about 50% by weight of the total composition, preferably 1% to about 30%, more preferably 10% to about 20%.

8. The orally disintegrating film composition according to claim 7, wherein the ratio of pregelatinized hydroxypropyl pea starch and pullulan is from 60:40 to 80:20, preferably from 65:35 to 75:25, especially from 67:33 to 73:27.

9. The orally disintegrating film composition of any one of claims 1 to 8, wherein the film composition has a disintegration time of 180 s or less, preferably of 120 s or less, especially of 60 s or less.

10. The orally disintegrating film composition of any one of claims 1 to 9, wherein the film composition has an ultimate tensile strength of 0.8 N/mm² or below, preferably of 0.5 N/mm² or below, especially of 0.3 N/mm² or below.

11. A method for producing an orally disintegrating film composition according to any one of claims 1 to 10, wherein a preparation comprising Buprenorphine is mixed with a preparation of the first film-forming polymer, preferably a starch, and a preparation of the second film-forming polymer, preferably pullulan, by stirring with at least 100 rpm for at least 10 min at a temperature of at least 40°C.

12. A method according to claim 11, wherein stirring is performed with at least 200 rpm for at least 30 min at a temperature of 40°C to 65°C, preferably of 45°C to 55°C.

13. A method according to claim 11 or 12, wherein the mixture is degassed after mixing for at least 6 h, preferably for at least 10 h.

14. A method according to any one of claims 11 to 13, wherein the mixture is casted and dried at a temperature of at least 60°C, preferably at a temperature of 65°C to 100°C.

15. A method according to any one of claims 11 to 14, wherein the mixture is casted and dried to a film with a thickness of 10 to 800 µm, preferably of 100 to 500 µm, especially of 130 to 200 µm.
